(19) Europäisches Patentamt
European Patent Office
Office européen des brevets

(11) Veröffentlichungsnummer: **0 520 277 A2**

# (12) EUROPÄISCHE PATENTANMELDUNG

(21) Anmeldenummer: **92110043.4**

(22) Anmeldetag: **15.06.92**

(51) Int. Cl.$^5$: **C07D 471/04**, C07D 487/04, A61K 31/47

(30) Priorität: **27.06.91 DE 4121214**

(43) Veröffentlichungstag der Anmeldung: **30.12.92 Patentblatt 92/53**

(84) Benannte Vertragsstaaten: **AT BE CH DE DK ES FR GB GR IT LI LU MC NL PT SE**

(71) Anmelder: **BAYER AG**
**W-5090 Leverkusen 1 Bayerwerk(DE)**

(72) Erfinder: **Petersen, Uwe, Dr.**
**Auf dem Forst 4**
**W-5090 Leverkusen(DE)**
Erfinder: **Krebs, Andreas, Dr.**
**Am Gartenfeld 70**
**W-5068 Odenthal-Holz(DE)**
Erfinder: **Schenke, Thomas, Dr.**
**Mühlenstrasse 113**
**W-5090 Bergisch Gladbach 2(DE)**
Erfinder: **Grohe, Klaus, Dr.**
**Am Wasserturm 10**
**W-5068 Odenthal(DE)**
Erfinder: **Bremm, Klaus-Dieter, Dr.**
**Wellinghofer Amtsstrasse 5**
**W-4600 Dortmund 30(DE)**
Erfinder: **Endermann, Rainer, Dr.**
**In den Birken 152a**
**W-5600 Wuppertal(DE)**
Erfinder: **Metzger, Karl-Georg, Dr.**
**Pahlkestrasse 75**
**W-5600 Wuppertal(DE)**
Erfinder: **Zeiler, Hans-Joachim, Dr.**
**Am Rohm 86**
**W-5600 Wuppertal(DE)**

## (54) 7-Azaisoindolinyl-chinolon- und naphthyridoncarbonsäure-Derivate.

(57) Die Erfindung betrifft neue Chinolon- und Naphthyridoncarbonsäure-Derivate, die in 7-Stellung durch einen gegebenenfalls partiell hydrierten Azaisoindolinylring substituiert sind, Verfahren zu ihrer Herstellung sowie diese enthaltende antibakterielle Mittel und Futterzusatzstoffe.

EP 0 520 277 A2

Die Erfindung betrifft neue Chinolon- und Naphthyridoncarbonsäure-Derivate, die in 7-Stellung durch einen gegebenenfalls partiell hydrierten Azaisoindolinylring substituiert sind, Verfahren zu ihrer Herstellung sowie diese enthaltende antibakterielle Mittel und Futterzusatzstoffe.

Es sind bereits aus der EP 343 560 Chinolon- und Naphthyridoncarbonsäuren bekannt geworden, die in 7-Stellung durch einen Isoindolinylring substituiert sind, wie z.B. 7-(2-Isoindolinyl)-1-cyclopropyl-6,8-difluor-1,4-dihydro-4-oxo-3-chinolincarbonsäure. Darüber hinaus sind aus der EP 424 850 1-Cyclopropyl-6,8-difluor-7-(3,8-diazabicyclo[4.3.0]non-1(6)-en-8-yl)-1,4-dihydro-4-oxo-chinolincarbonsäure und aus der EP 424 851 9-Fluor-3(S)-methyl-10-(3,8-diazabicyclo[4.3.0]non-1(6)-en-8-yl)-7-oxo-2,3-dihydro-7H-pyrido[1,2,3-de]-[1,4]benzoxazin-6-carbonsäure bekanntgeworden. Die antibakterielle Wirksamkeit dieser Verbindungen weist jedoch Lücken auf.

Es wurde gefunden, daß die Verbindungen der Formel (I)

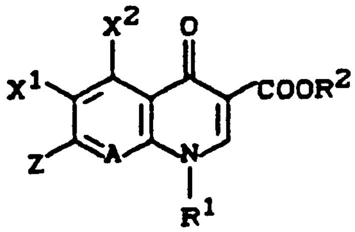

(I),

in welcher

- $X^1$ für Halogen,
- $X^2$ für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen, Arylthio, Halogen oder Methyl,
- $R^1$ für Alkyl mit 1 bis 4 Kohlenstoffatomen, Alkenyl mit 2 bis 4 Kohlenstoffatomen, Cycloalkyl mit 3 bis 6 Kohlenstoffatomen, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,
- $R^2$ für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-methyl,
- Z für einen Rest der Struktur

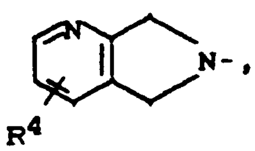
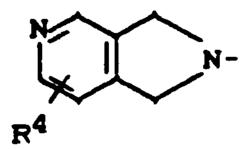
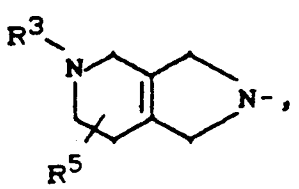
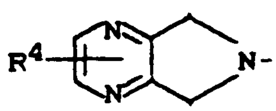

steht, worin

- $R^3$ für Wasserstoff, gegebenenfalls durch Hydroxy substituiertes $C_1$-$C_3$-Alkyl, Alkoxycarbonyl mit 1 bis 4 C-Atomen im Alkoxyteil oder $C_1$-$C_3$-Acyl,
- $R^4$ für Wasserstoff, Hydroxy,

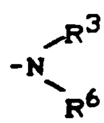

,

Hydroxymethyl oder

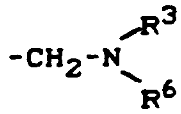

steht, wobei

$R^6$ Wasserstoff oder Methyl bedeutet,

$R^5$ für Wasserstoff, $C_1$-$C_3$-Alkyl oder Cyclopropyl und

A für N oder C-$R^7$ steht, worin

$R^7$ für H, Halogen, Methyl, Hydroxy oder Methoxy steht oder auch gemeinsam mit $R^1$ eine Brücke der Struktur

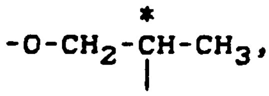

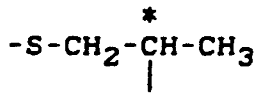

oder

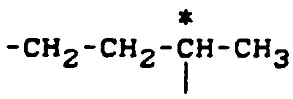

bilden kann, mit der Maßgabe, daß A nicht N, CH oder CF sein kann und mit $R^1$ keine Brücke der Struktur

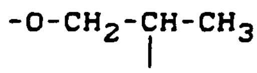

bilden kann, wenn

Z für

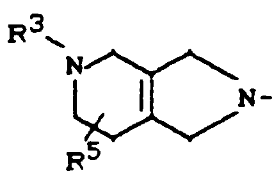

steht,

und deren pharmazeutisch verwendbaren Hydrate und Säureadditionssalze sowie die Alkali-, Erdalkali-, Silber- und Guanidiniumsalze der zugrundeliegenden Carbonsäuren gegenüber dem Stand der Technik eine höhere antibakterielle Wirkung insbesondere im grampositiven Bereich aufweisen.

Bevorzugt sind die Verbindungen der Formel (I),

in welcher

$X^1$ für Fluor,

$X^2$ für Wasserstoff, Amino, Methylamino, Hydroxy, Methoxy, Fluor, Chlor, Brom oder Methyl,

$R^1$ für Alkyl mit 1 bis 3 Kohlenstoffatomen, Vinyl, Cycloalkyl mit 3 bis 4 Kohlenstoffatomen, 2-Fluorethyl, gegebenenfalls durch 1 oder 2 Fluoratome substituiertes Phenyl,

$R^2$ für Wasserstoff, gegebenenfalls durch Amino, Methylamino oder Dimethylamino substituiertes Alkyl mit 1 bis 2 Kohlenstoffatomen oder (5-Methyl-2-oxo-1,3-dioxol-4-yl)-methyl,

Z für einen Rest der Struktur

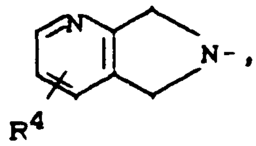

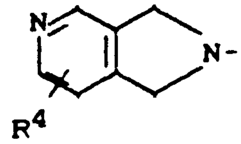

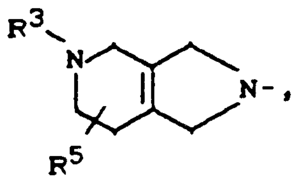

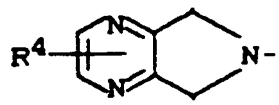

steht, worin

$R^3$ für Wasserstoff, gegebenenfalls durch Hydroxy substituiertes $C_1$-$C_2$-Alkyl, Alkoxycarbonyl mit 1 bis 4 C-Atomen im Alkoxyteil oder $C_1$-$C_3$-Acyl,

$R^4$ für Wasserstoff, Hydroxy oder

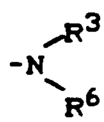

steht, wobei

$R^6$ Wasserstoff oder Methyl bedeutet,

$R^5$ für Wasserstoff oder Methyl und

A für N oder C-$R^7$ steht, worin

$R^7$ für H, Fluor, Chlor, Brom, Methyl oder Methoxy steht oder auch gemeinsam mit $R^1$ eine Brücke der Struktur

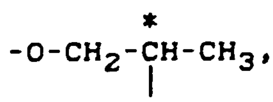

bilden kann, mit der Maßgabe, daß A nicht N, CH oder CF sein kann und mit $R^1$ keine Brücke der Struktur

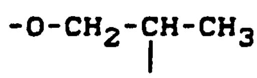

bilden kann, wenn

Z für

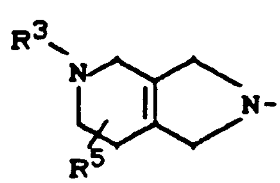

steht,

und deren pharmazeutisch verwendbare Hydrate und Säureadditionssalze sowie die Alkali-,

Erdalkali-, Silber- und Guanidiniumsalze der zugrundeliegenden Carbonsäuren.

Besonders bevorzugt sind die Verbindungen der Formel (I),

in welcher

- $X^1$ für Fluor,
- $X^2$ für Wasserstoff, Amino, Fluor oder Brom,
- $R^1$ für Alkyl mit 1 bis 2 Kohlenstoffatomen, Cyclopropyl, gegebenenfalls durch 1 oder 2 Fluoratome substituiertes Phenyl,
- $R^2$ für Wasserstoff oder Alkyl mit 1 bis 2 Kohlenstoffatomen,
- Z für einen Rest der Struktur

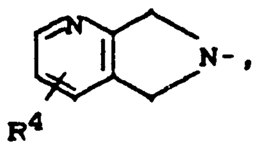

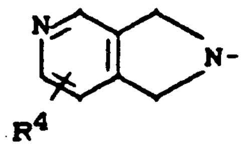

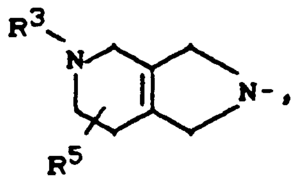

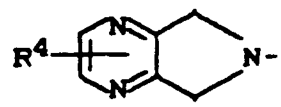

steht, worin

- $R^3$ für Wasserstoff, Methyl, Alkoxycarbonyl mit 1 bis 4 C-Atomen im Alkoxyteil oder $C_1$-$C_3$-Acyl,
- $R^4$ für Wasserstoff, Hydroxy oder

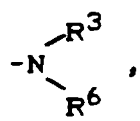

steht, wobei

- $R^6$ Wasserstoff oder Methyl bedeutet,
- $R^5$ für Wasserstoff oder Methyl und
- A für N oder C-$R^7$ steht, worin
- $R^7$ für H, Fluor, Chlor oder Methoxy steht oder auch gemeinsam mit $R^1$ eine Brücke der Struktur

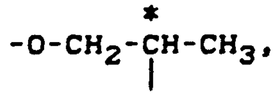

bilden kann, mit der Maßgabe, daß A nicht N, CH oder CF sein kann und mit $R^1$ keine Brücke der Struktur

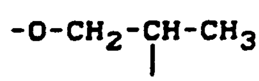

bilden kann, wenn

Z für

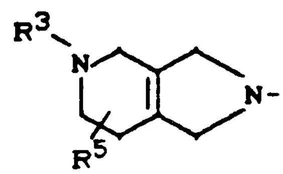

steht,

und deren pharmazeutisch verwendbaren Hydrate und Säureadditionssalze sowie die Alkali-, Erdalkali-, Silber- und Guanidiniumsalze der zugrundeliegenden Carbonsäuren.

Weiterhin wurde gefunden, daß man die Verbindungen der Formel (I) erhält, wenn man eine Verbindung der Formel (II)

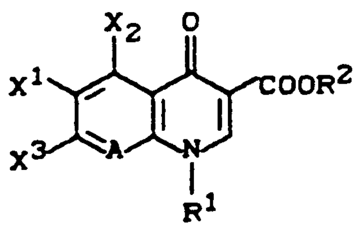

(II),

in welcher

A, $R^1$, $R^2$, $X^1$ und $X^2$ die oben angegebene Bedeutung haben und

$X^3$ für Halogen, insbesondere Fluor oder Chlor steht,

mit Verbindungen der Formel (III)

Z-H (III)

in welcher

Z die oben angegebene Bedeutung hat,

gegebenenfalls in Gegenwart von Säurefängern umsetzt.

Verwendet man beispielsweise 8-Chlor-1-cyclopropyl-6,7-difluor-1,4-dihydro-4-oxo-3-chinolincarbonsäure und 5-Methyl-2,3,4,5,6,7-hexahydro-1H-pyrrolo[3,4-c]pyridin als Ausgangsstoffe, so kann der Reaktionsverlauf durch folgendes Formelschema wiedergegeben werden:

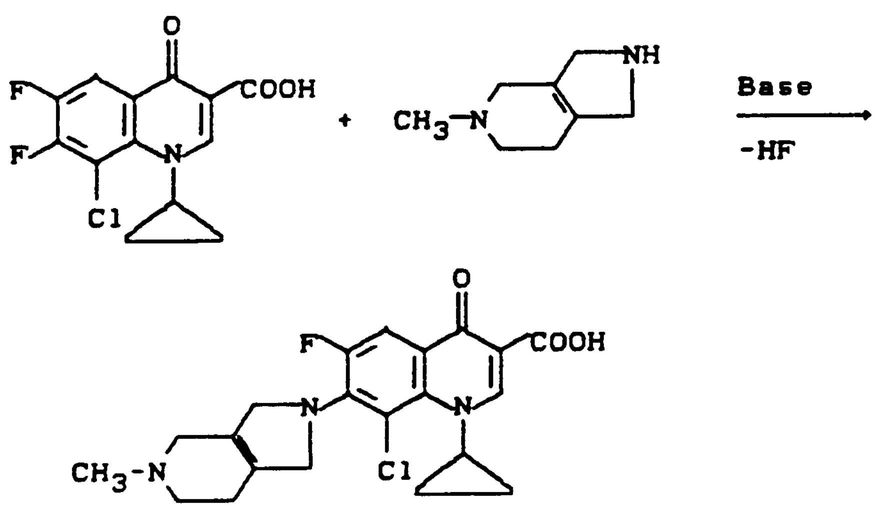


Die meisten der als Ausgangsstoffe verwendeten Verbindungen der Formel (II) sind bekannt oder können nach bekannten Methoden hergestellt werden. Als Beispiele seien genannt:

7-Chlor-1-cyclopropyl-6-fluor-1,4-dihydro-4-oxo-3-chinolincarbonsäure (Deutsche Patentanmeldung 3 142 854),

1-Cyclopropyl-6,7-difluor-1,4-dihydro-4-oxo-3-chinolincarbonsäure (Europäische Patentanmeldung 113 091),
6-Chlor-1-cyclopropyl-7,8-difluor-1,4-dihydro-4-oxo-3-chinolincarbonsäure (Deutsche Patentanmeldung 3 420 743),
8-Chlor-1-cyclopropyl-6,7-difluor-1,4-dihydro-4-oxo-3-chinolincarbonsäure (Deutsche Patentanmeldung 3 420 743),
1-Cyclopropyl-6,7,8-trifluor-1,4-dihydro-4-oxo-3-chinolincarbonsäure (Deutsche Patentanmeldung 3 318 145),
5-Brom-1-cyclopropyl-6,7,8-trifluor-1,4-dihydro-4-oxo-3-chinolincarbonsäure,
5-Brom-1-(2,4-difluorphenyl)-6,7,8-trifluor-1,4-dihydro-4-oxo-3-chinolincarbonsäure,
1-Cyclopropyl-6,7-difluor-1,4-dihydro-8-methyl-4-oxo-3-chinolincarbonsäure,
6,7-Difluor-1-ethyl-1,4-dihydro-4-oxo-3-chinolincarbonsäure,
7-Chlor-6-fluor-1-ethyl-1,4-dihydro-4-oxo-3-chinolincarbonsäure,
7-Chlor-6-fluor-1,4-dihydro-1-(2-hydroxyethyl)-4-oxo-3-chinolincarbonsäure,
6,7-Difluor-1-(2-fluorethyl)-1,4-dihydro-4-oxo-3-chinolincarbonsäure,
7-Chlor-6-fluor-1,4-dihydro-1-methoxy-4-oxo-3-chinolincarbonsäure,
7-Chlor-6-fluor-1,4-dihydro-1-methylamino-4-oxo-3-chinolincarbonsäure,
6,7-Difluor-1,4-dihydro-4-oxo-1-phenyl-3-chinolincarbonsäure,
7-Chlor-1-cyclopropyl-6-fluor-1,4-dihydro-4-oxo-1,8-naphthyridin-3-carbonsäure,
7-Chlor-1-cyclopropyl-6-fluor-1,4-dihydro-4-oxo-1,8-naphthyridin-3-carbonsäureethylester,
1-Cyclopropyl-6,7,8-trifluor-1,4-dihydro-4-oxo-3-chinolincarbonsäureethylester (Deutsche Patentanmeldung 3 318 145),
9,10-Difluor-2,3-dihydro-3-methyl-7-oxo-7H-pyrido[1,2,3-de][1,4]benzoxacin-6-carbonsäure (Europäische Patentanmeldung 47 005),
8,9-Difluor-6,7-dihydro-5-methyl-1-oxo-1H,5H-benzo[i,j]chinolicin-2-carbonsäure,
7-Chlor-6-fluor-1-phenyl-1,4-dihydro-4-oxo-1,8-naphthyridin-3-carbonsäure (Europäische Patentanmeldung 153 580),
7-Chlor-6-fluor-1-(4-fluorphenyl)-1,4-dihydro-4-oxo-1,8-naphthydrin-3-carbonsäureethylester,
6,7,8-Trifluor-1,4-dihydro-1-methylamino-4-oxo-3-chinolincarbonsäure (Deutsche Patentanmeldung 3 409 922),
1-Amino-6,7,8-trifluor-1,4-dihydro-4-oxo-3-chinolincarbonsäure (Deutsche Patentanmeldung 3 409 922),
6,7,8-Trifluor-1,4-dihydro-1-dimethylamino-4-oxo-3-chinolincarbonsäure (Deutsche Patentanmeldung 3 409 922),
6,7-Difluor-1-(4-fluorphenyl)-1,4-dihydro-8-methyl-4-oxo-3-chinolincarbonsäure,
7-Chlor-6-fluor-1-(4-fluorphenyl)-1,4-dihydro-4-oxo-3-chinolincarbonsäure (Europäische Patentanmeldung 131 839),
7-Chlor-6-fluor-1-(2,4-difluorphenyl)-1,4-dihydro-4-oxo-3-chinolincarbonsäure (Europäische Patentanmeldung 131 839),
6,7,8-Trlfluor-1-(4-fluorphenyl)-1,4-dihydro-4-oxo-3-chinolincarbonsäure (Europäische Patentanmeldung 154 780),
6,7,8-Trifluor-1-(2,4-difluorphenyl)-1,4-dihydro-4-oxo-3-chinolincarbonsäure (Europäische Patentanmeldung 154 780),
6,7,8-Trifluor-1,4-dihydro-4-oxo-1-phenyl-3-chinolincarbonsäure (Europäische Patentanmeldung 154 780),
7-Chlor-1-ethyl-6-fluor-1,4-dihydro-4-oxo-1,8-naphthyridin-3-carbonsäure,
6,7-Difluor-1,4-dihydro-4-oxo-1-vinyl-3-chinolincarbonsäure,
1-Cyclopropyl-5,6,7,8-tetrafluor-1,4-dihydro-4-oxo-3-chinolincarbonsäure,
5-Amino-1-cyclopropyl-6,7,8-trifluor-1,4-dihydro-4-oxo-3-chinolincarbonsäure,
1-Cyclopropyl-6,7,8-trifluor-1,4-dihydro-5-hydroxy-4-oxo-3-chinolincarbonsäure,
1-Cyclopropyl-6,7-difluor-1,4-dihydro-8-methoxy-4-oxo-3-chinolincarbonsäure,
7-Chlor-1-(2,4-difluorphenyl)-6-fluor-1,4-dihydro-4-oxo-1,8-naphthyridin-3-carbonsäure,
7-Chlor-1-(2,4-difluorphenyl)-6-fluor-1,4-dihydro-4-oxo-1,8-naphthyridin-3-carbonsäureethylester.

Die als Ausgangsverbindungen benötigten bicyclischen Amine der Formel (III) sind teilweise neu. Sie können nach folgenden Verfahren hergestellt werden.

1) N-Propargylurethan kann in Gegenwart starker Basen mit 5- oder 2-Chlormethylpyrimidin oder Chlormethylpyrazin alkyliert werden. Die sich anschließende intramolekulare Hetero-Diels-Alder-Reaktion erfolgt entweder thermisch oder protonenkatalysiert [Tetrahedron 45, 6519 (1989)].

$HC{\equiv}CH-CH_2-NHCOOC_2H_5$

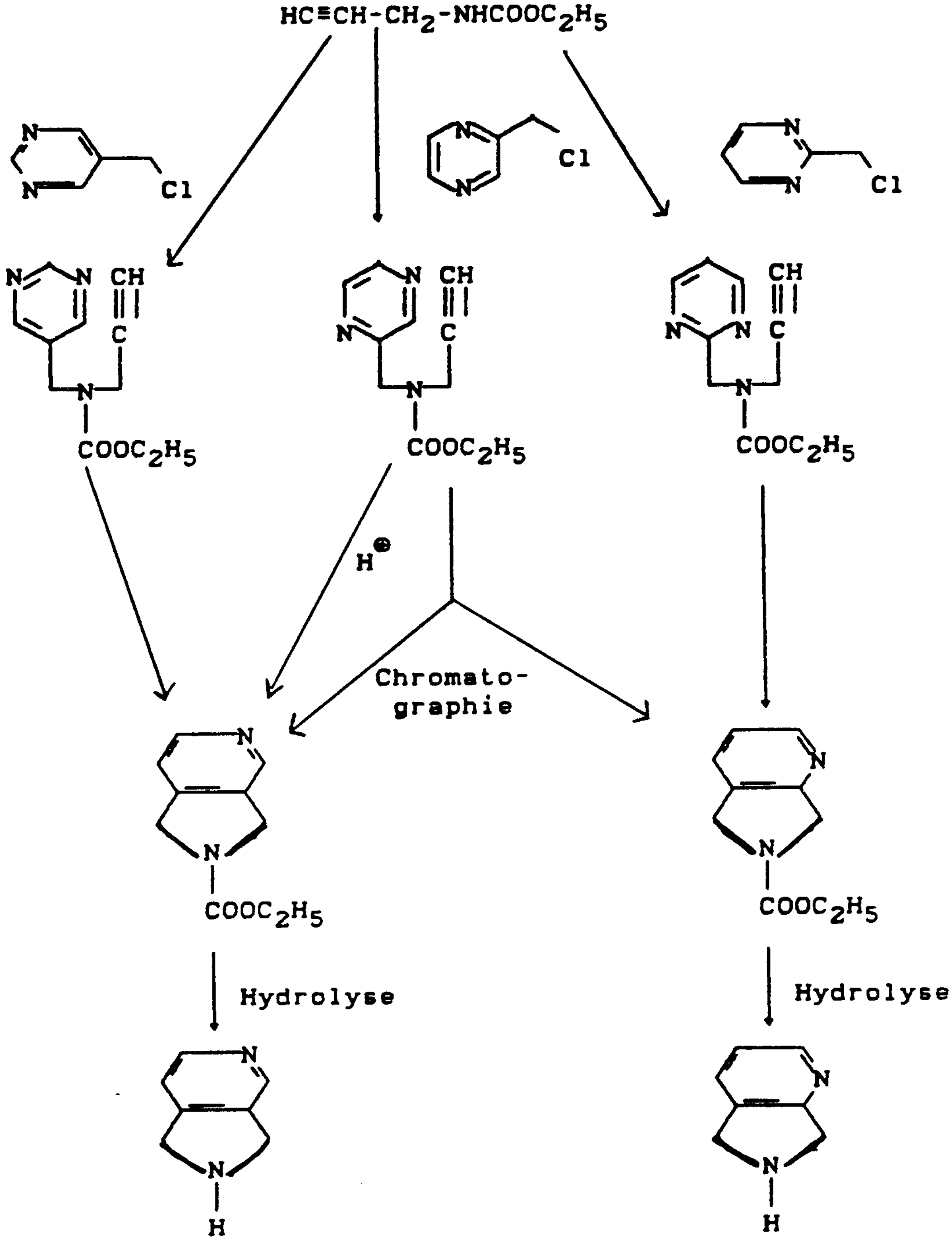


Die Zwischenstufen der N-Propargyl-N-pyrazinylmethylurethane beziehungsweise N-Propargyl-N-(2-pyrimidinylmethyl)urethane und N-Propargyl-N-(5-pyrimidinylmethyl)-urethane können auch durch Acylieren von Aminomethylpyrazin, 2-Aminomethylpyrimidin oder 5-Aminomethylpyrimidin mit Chlorameisensäureestern und anschließender Alkylierung mit Propargylhalogeniden erhalten werden.

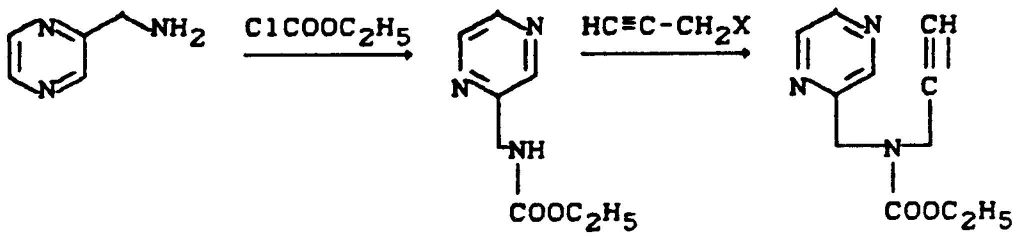


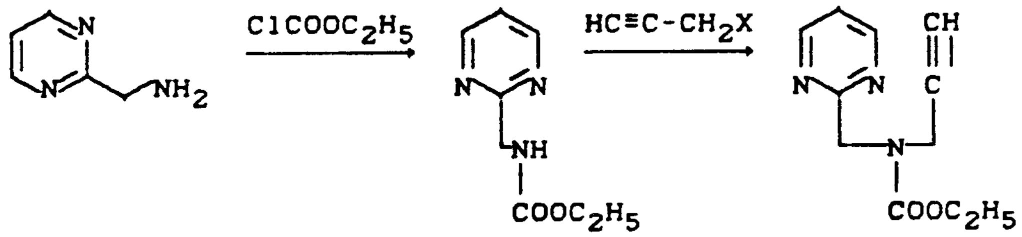


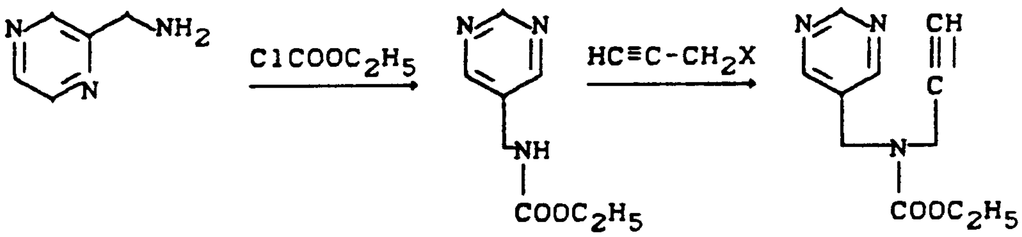


2) Die aus den bekannten Pyrrolidin-3-onen nach Standardverfahren herstellbaren Enamine können mit 1,2,4-Triazin in einer Hetero-Diels-Alder-Reaktion umgesetzt werden [Tetrahedron 39, 2869 (1983)].

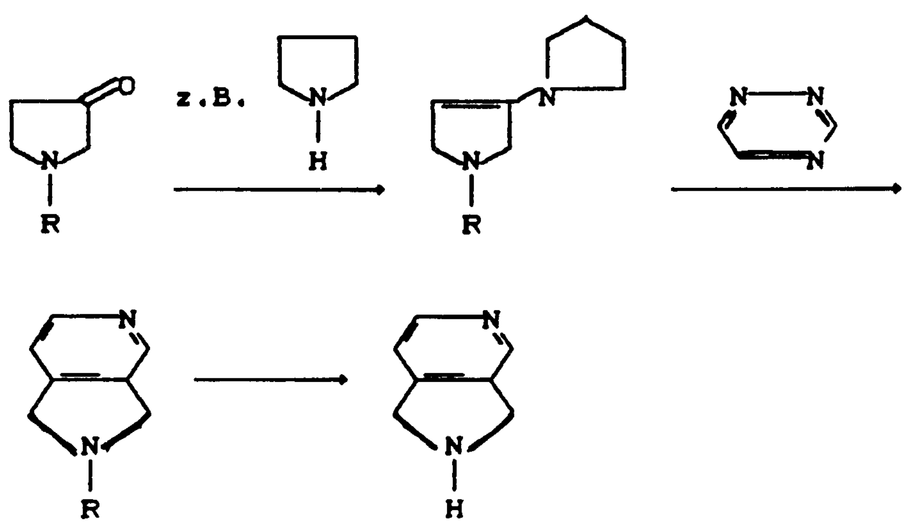


R = Acyl, Benzyl

3) Pyrrolidin-3-one können auch in die O-Allyloximether überführt werden, die durch Erhitzen in die Pyridinderivate umgelagert werden können [Synthesis 1979, 221):

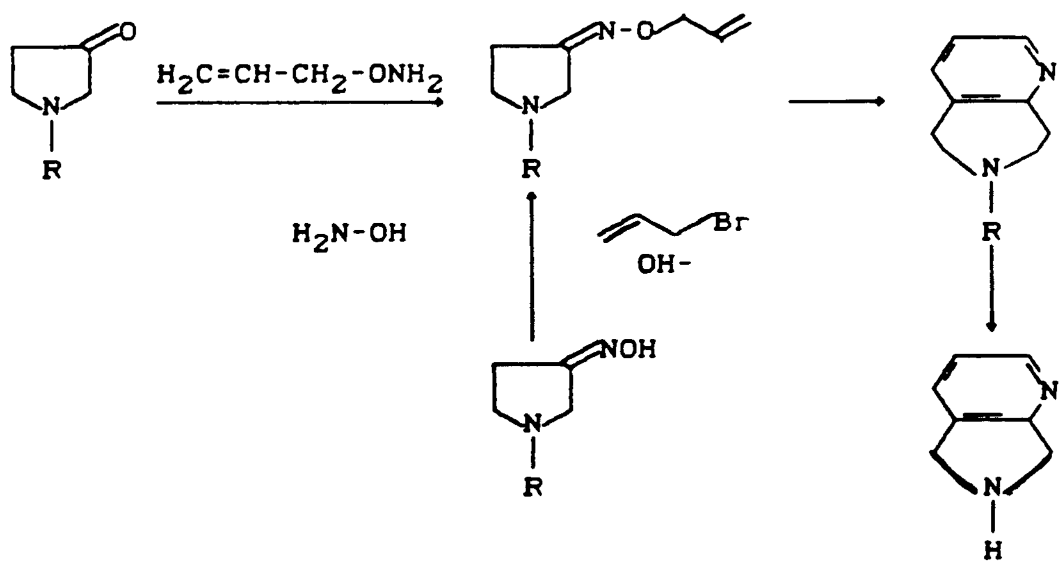


R = Acyl, Benzyl

4) Pyrrolidin-3-one können auch mit 3-Aminoacrolein zu den Pyridinderivaten umgesetzt werden (Tetrahedron Lett. 1970, 3291).

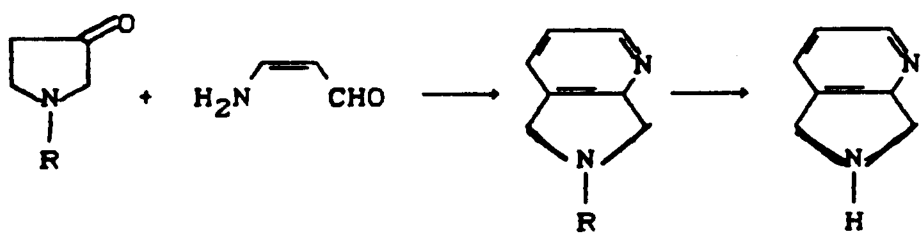

R = Acyl, Benzyl

5) Aminosubstituierte Dihydropyrrolopyridine können durch Reduktion der entsprechenden Nitroverbindungen, die auf verschiedenen Wegen zugänglich sind, erhalten werden:

a) Die Enamine der literaturbekannten Pyrrolidin-3-one können mit 5-Nitropyrimidin eine Hetero-Diels-Alder-Reaktion eingehen [Tetrahedron 39, 2869 (1983)].

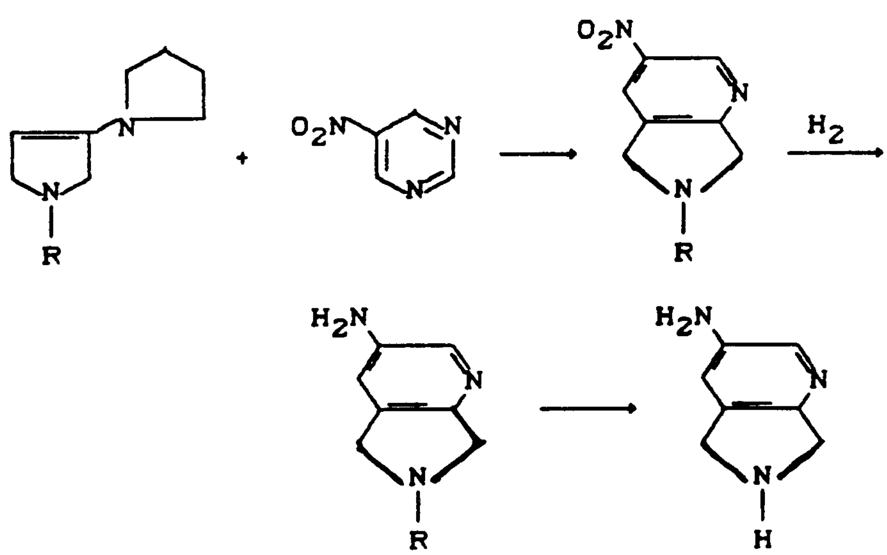

R = Acyl, Benzyl

b) Die Dihydropyrrolopyridine können in die N-Oxyde überführt und dann nitriert werden.

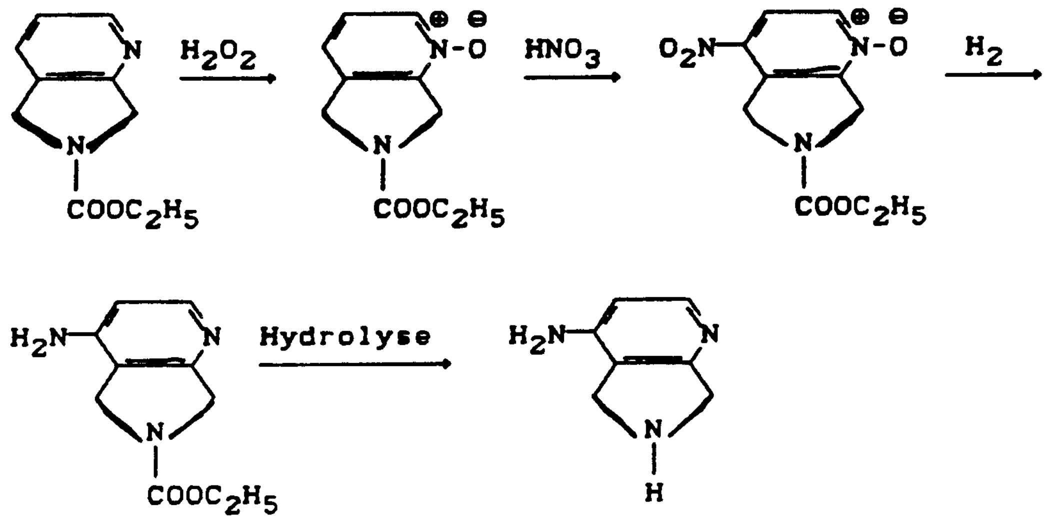


Die so erhaltenen Aminoverbindungen können nach allgemein bekannten Methoden einmal oder zweimal an der Aminogruppe alkyliert werden.

6) Durch Alkylieren der Dihydropyrrolopyridine und Reduktion der so erhaltenen Pyridiniumsalze mit Natriumborhydrid können die entsprechenden Hexahydroderivate hergestellt werden:

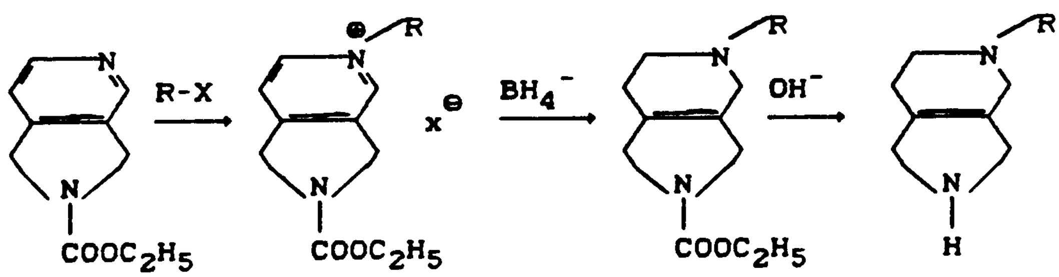


R = Alkyl, X = Halogen, Sulfonat

7) 2,3-Bis-(chlormethyl)-pyrazin [Synthesis, 676 (1984)] kann mit Benzylamin oder Amiden zum 2,3-Dihydro-1H-pyrolo[3,4-b]pyrazin umgesetzt werden. Die Benzylschutzgruppe kann abhydriert, die Acylschutzgruppen können durch saure oder basische Hydrolyse abgespalten werden.

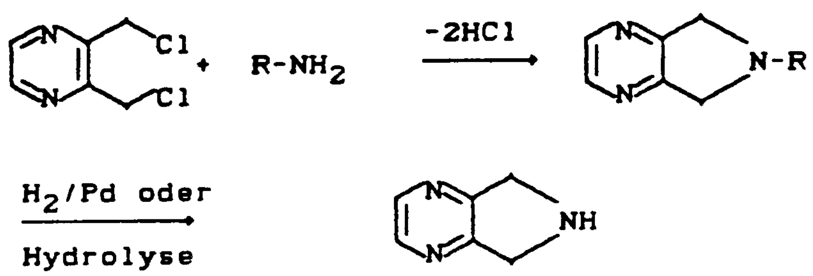


R = Benzyl, Acyl

Als Beispiele für Verbindungen der Formel (III) seien genannt:

2,3,4,5,6,7-Hexahydro-1H-pyrrolo[3,4-c]pyridin,

5-Methyl-2,3,4,5,6,7-hexahydro-1H-pyrrolo[3,4-c]pyridin,

5-Ethyl-2,3,4,5,6,7-hexahydro-1H-pyrrolo[3,4-c]pyridin,

5-(2-Hydroxyethyl)-2,3,4,5,6,7-hexahydro-1H-pyrrolo[3,4-c]pyridin,

5-(tert.-Butoxycarbonyl)-2,3,4,5,6,7-hexahydro-1H-pyrrolo[3,4-c]pyridin,
2,3-Dihydro-1H-pyrrolo[3,4-b]pyrazin.

Die Umsetzung von (II) mit (III), bei der die Verbindungen (III) auch in Form ihrer Salze, wie z.B. der Hydrochloride eingesetzt werden können, wird vorzugsweise in einem Verdünnungsmittel wie Dimethylsulfoxid, N,N-Dimethylformamid, N-Methylpyrrolidon, Hexamethyl-phosphorsäuretrisamid, Sulfolan, Acetonitril, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2.2.2]octan (DABCO), 1,8-Diazabicyclo[5.4.0]undec-7-en (DBU) oder überschüssiges Amin (III).

Die Reaktionstemperaturen können in einem größeren Bereich variiert werden. Im allgemeinen arbeitet man zwischen etwa 20 und 200 °C, vorzugsweise zwischen 80 und 180 °C.

Die Umsetzung kann bei Normaldruck, aber auch bei erhöhtem Druck durchgeführt werden. Im allgemeinen arbeitet man bei Drucken zwischen etwa 1 und 100 bar, vorzugsweise zwischen 1 und 10 bar.

Bei der Durchführung des erfindungsgemäßen Verfahrens setzt man auf 1 Mol der Verbindung (II) 1 bis 15 Mol, vorzugsweise 1 bis 6 Mol, der Verbindung (III) ein.

Freie Aminogruppen können während der Umsetzung durch eine geeignete Aminoschutzgruppe, z.B. durch den tert.-Butoxycarbonylrest oder als Azomethingruppe, geschützt und nach Beendigung der Reaktion durch Behandlung mit einer geeigneten Säure wie Chlorwasserstoffsäure oder Trifluoressigsäure wieder freigesetzt werden (siehe Houben-Weyl, Methoden der Organischen Chemie, Band E4, Seite 144 (1983); J.F.W. McOmie, Protective Groups in Organic Chemistry (1973), Seite 43).

Zur Herstellung der erfindungsgemäßen Ester wird die zugrundeliegende Carbonsäure vorzugsweise in überschüssigem Alkohol in Gegenwart von starken Säuren, wie Schwefelsäure, wasserfreiem Chlorwasserstoff, Methansulfonsäure, p-Toluolsulfonsäure oder sauren Ionenaustauschern, bei Temperaturen von etwa 20 bis 200 °C, vorzugsweise etwa 60 bis 120 °C, umgesetzt. Das entstehende Reaktionswasser kann auch durch azeotrope Destillation mit Chlorofom, Tetrachlormethan, Benzol oder Toluol entfernt werden.

Die Herstellung von Estern gelingt auch vorteilhaft durch Erhitzen der zugrundeliegenden Säure mit Dimethylformamiddialkylacetat in einem Lösungsmittel wie Dimethylformamid.

Die als Prodrug verwendeten (5-Methyl-2-oxo-1,3-dioxol-4-yl-methyl)-ester werden durch Umsetzung eines Alkalisalzes der zugrundeliegenden Carbonsäure, die gegebenenfalls am N-Atom durch eine Schutzgruppe wie den tert.-Butoxycarbonylrest geschützt sein kann, mit 4-Brommethyl- oder 4-Chlormethyl-5-methyl-1,3-dioxol-2-on in einem Lösungsmittel wie Dimethylformamid, Dimethylacetamid, N-Methylpyrrolidon, Dimethylsulfoxid oder Tetramethylharnstoff bei Temperaturen von etwa 0 bis 100 °C, vorzugsweise 0 bis 50 °C, erhalten.

Die Herstellung der Säureadditionssalze der erfindungsgemäßen Verbindungen erfolgt in üblicher Weise zum Beispiel durch Lösen des Betains in überschüssiger wäßriger Säure und Ausfällen des Salzes mit einem mit Wasser mischbaren organischen Lösungsmittel wie Methanol, Ethanol, Aceton, Acetonitril. Man kann auch äquivalente Mengen Betain und Säure in Wasser oder einem Alkohol wie Glykolmonomethylether erhitzen und anschließend bis zur Trockne eindampfen oder das ausgefallene Salz absaugen. Als pharmazeutisch verwendbare Salze sind beispielsweise die Salze der Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäure, Embonsäure, Glutaminsäure oder Asparaginsäure zu verstehen.

Die Alkali- oder Erdalkalisalze der erfindungsgemäßen Carbonsäuren werden beispielsweise durch Lösen des Betains in unterschüssiger Alkali- oder Erdalkalilauge, Filtration von ungelöstem Betain und Eindampfen des Filtrats bis zur Trockne erhalten. Pharmazeutisch geeignet sind Natrium-, Kalium- oder Calciumsalze. Durch Umsetzung eines Alkali- oder Erdalkalisalzes mit einem geeigneten Silbersalz wie Silbernitrat werden die entsprechenden Silbersalze erhalten.

Außer den in den Beispielen genannten Wirkstoffen können auch die in der folgenden Tabelle aufgeführten Wirkstoffe hergestellt werden:

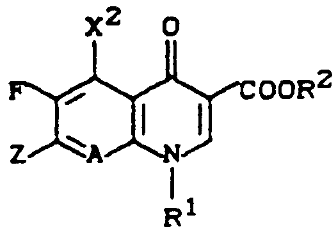

| $R^1$ | $R^2$ | $X^2$ | Z | A |
|---|---|---|---|---|
| 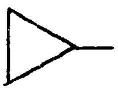 | H | H | 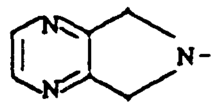 | $C-OCH_3$ |
| 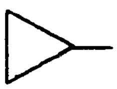 | H | H | 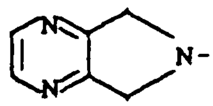 | CF |
| 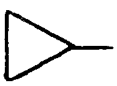 | H | H | 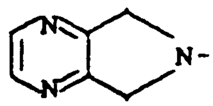 | CH |
| 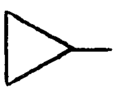 | H | H | 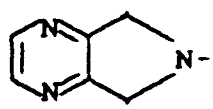 | CCl |

| $R^1$ | $R^2$ | $X^2$ | Z | A |
|---|---|---|---|---|
| 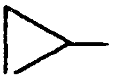 | H | H | 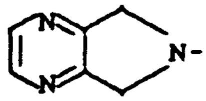 | N |
| 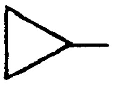 | H | $NH_2$ | 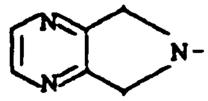 | CF |
| 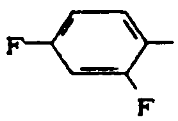 | H | H | 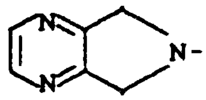 | N |
| $(CH_3)_3C-$ | H | H | 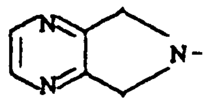 | CH |
| 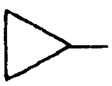 | H | H | 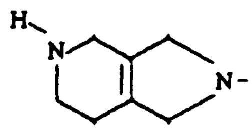 | CCl |
| 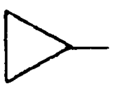 | H | H | 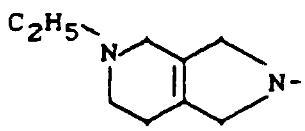 | CCl |
| 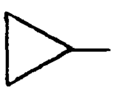 | H | Cl | 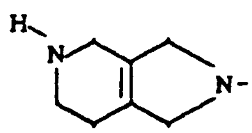 | CCl |
| 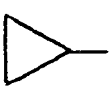 | H | Cl | 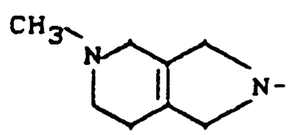 | CCl |

| $R^1$ | $R^2$ | $X^2$ | Z | A |
|---|---|---|---|---|
| 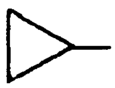 | H | H | 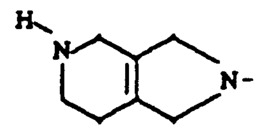 | $C-CH_3$ |
| 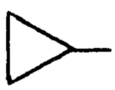 | H | H | 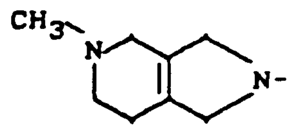 | $C-CH_3$ |
| 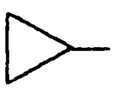 | H | H | 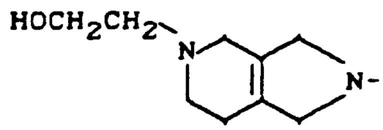 | CCl |
| 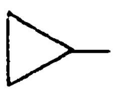 | H | H | 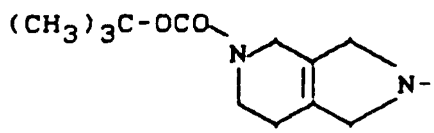 | CCl |
| 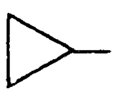 | H | H | 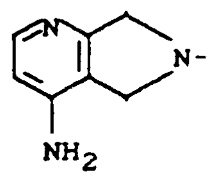 | CF |
| 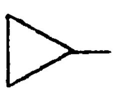 | H | H | 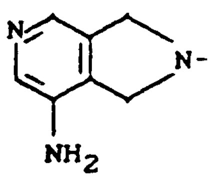 | CF |
| 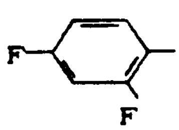 | H | H | 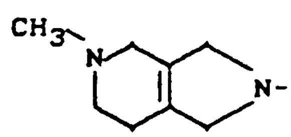 | CCl |

| $R^1$ | $R^2$ | $X^2$ | Z | A |
|---|---|---|---|---|
| 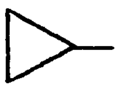 | $-CH_2CH_2NH_2$ | H | 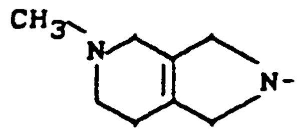 | CCl |
| 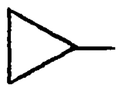 | H | H | 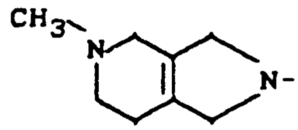 | $OCH_3$ |

Die erfindungsgemäßen Verbindungen wirken stark antibiotisch und zeigen bei geringer Toxizität ein breites antibakterielles Spektrum gegen grampositive und gramnegative Keime, insbesondere gegen Enterobakterien; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gramnegative und grampositive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Die Verbindungen eignen sich ferner zur Bekämpfung von Protozoonosen und Helminthosen.

Die erfindungsgemäßen Verbindungen können in verschiedenen pharmazeutischen Zubereitungen angewendet werden. Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Die minimalen Hemmkonzentrationen (MHK) wurden per Reihenverdünnungsverfahren auf Iso-Sensitest Agar (Oxoid) bestimmt. Für jede Prüfungssubstanz wurde eine Reihe von Agarplatten hergestellt, die bei jeweils doppelter Verdünnung abfallende Konzentrationen des Wirkstoffes enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt ca. $10^4$ koloniebildende Partikel enthielt. Die beimpften Agarplatten wurden bei 37°C bebrütet, und das Keimwachstum wurde nach ca. 20 Stunden abgelesen. Der MHK-Wert (µg/ml) gibt die niedrigste Wirkstoffkonzentration an, bei der mit bloßem Auge kein Wachstum zu erkennen war.

In der nachstehenden Tabelle sind die MHK-Werte einiger der erfindungsgemäßen Verbindungen im Vergleich 7-(4-Amino-1,3-dihydro-isoindol-2-yl)-1-cyclopropyl-6,8-difluor-1,4-dihydro-4-oxo-3-chinolincarbonsäure (EP 343 560, Beispiel 2) aufgeführt.

Tabelle: MHK-Werte

| Teststamm: | Beispiel | 1B | 2 | 3 | 5 | 6 | 4 | 7B | 8 | 9 | Ref.* |
|---|---|---|---|---|---|---|---|---|---|---|---|
| E.coli | Neumann 455/7 | 0,13<br>8 | 0,06<br>8 | 0,13<br>128 | 0,13<br>128 | 0,13<br>128 | 0,06<br>128 | 0,02<br>2 | 0,03<br>1 | 0,25<br>128 | 0,5<br>128 |
| Klebsiella sp. | 8085 | 0,25 | 0,13 | 0,5 | 1 | 0,5 | 0,25 | 0,06 | 0,06 | 0,5 | 4 |
| Morganella morg. | 932 | 0,13 | 0,13 | 0,25 | 0,13 | 0,25 | 0,5 | 0,03 | 0,06 | 0,5 | 1 |
| Providencia spp. | 12012 | 0,25 | 0,25 | 0,5 | 0,5 | 1 | 0,5 | 0,13 | 0,13 | 0,5 | 1 |
| Staphylococcus aureus | 1756 | 0,02 | 0,06 | 0,25 | 0,13 | 0,25 | 0,06 | 0,06 | 0,03 | 0,06 | 0,03 |
| | 133 | 0,02 | 0,25 | 0,25 | 0,13 | 0,25 | 0,06 | 0,06 | 0,03 | 0,06 | 0,03 |
| Enterococcus faecalis | 27101 | 0,01 | 0,25 | 0,5 | 0,25 | 0,5 | 0,25 | 0,13 | 0,06 | 0,5 | 0,5 |
| | 9790 | 0,06 | 0,13 | 0,5 | 0,25 | 0,5 | 0,25 | 0,13 | 0,06 | 0,5 | 1 |
| Pseudomonas aeruginosa | Walter | 4 | 1 | 2 | 128 | 16 | 4 | 1 | 0,5 | 128 | 128 |

*) Referenzverbindung:

7-(4-Amino-1,3-dihydro-isoindol-2-yl)-1-cyclopropyl-6,8-difluor-1,4-dihydro-4-oxo-3-chinolincarbonsäure (EP 343 560, Beispiel 2)

Beispiel Z1

5,7-Dihydro-6H-pyrrolo[3,4-b]pyridin

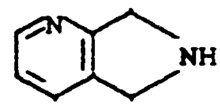

a) N-Propargylcarbamidsäureethylester

Man legt 115 g (2,1 mol) Propargylamin in 1 l Toluol vor, setzt 91 g Natriumhydroxid in 400 ml Wasser gelöst hinzu und tropft bei 10°C 239 g (2,2 mol) Chlorameisensäureethylester hinzu. Man rührt drei Stunden bei Raumtemperatur, trennt die organische Phase ab, extrahiert die wäßrige Phase mit Toluol, trocknet die organischen Lösungen über Magnesiumsulfat, engt ein und destilliert.

Ausbeute: 221 g (83 % der Theorie)

Siedepunkt: 101°C/20 mbar

b) 2-(N-Ethoxycarbonyl-N-propargylaminomethyl)-pyrimidin

Man legt 11,5 g (91 mmol) N-Propargylcarbamidsäureethylester in 90 ml Toluol vor, setzt 20,3 g KOH-Pulver und 0,5 g Triethylbenzylammoniumchlorid hinzu und tropft bei Raumtemperatur 13,5 g (104 mmol) 2-Chlormethylpyrimidin (DOS 2 932 643) hinzu. Man rührt über Nacht bei Raumtemperatur, saugt die Salze ab, wäscht das Filtrat mit gesättigter Kochsalzlösung, trocknet es über Magnesiumsulfat, engt ein und destilliert.

Ausbeute: 10,4 g (51 % der Theorie)

Siedepunkt: 130°C/0,35 mbar

c) 5,7-Dihydro-6H-pyrrolo[3,4-b]pyridin-6-carbonsäureethylester

Man erhitzt 7,7 g (35 mmol) 2-(N-Ethoxycarbonyl-N-propargylaminomethyl)-pyrimidin in 150 ml Xylol 40 Stunden unter Rückfluß. Man engt ein und kristallisiert den Rückstand aus Waschbenzin um.

Ausbeute: 5,5 g (81,7 % der Theorie)

Schmelzpunkt: 77 - 79°C

d) 5,7-Dihydro-6H-pyrrolo[3,4-b]pyridin-Dihydrochlorid

Man erhitzt 8,5 g (44 mmol) 5,7-Dihydro-1H-pyrrolo[3,4-b]pyridin-6-carbonsäureethylester in 90 ml konzentrierter Salzsäure über Nacht unter Rückfluß. Man engt die Lösung ein, verrührt den Rückstand mit Aceton, saugt das Salz ab und trocknet an der Luft.

Ausbeute: 7,5 g (88 % der Theorie)

Beispiel Z2

2,3-Dihydro-1H-pyrrolo[3,4-c]pyridin

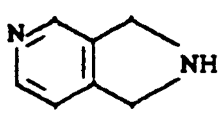

a) (N-Ethoxycarbonyl-N-propargyl)-aminomethylpyrazin

Man legt 14 g (0,11 mol) N-Propargylurethan in 110 ml Toluol vor, setzt 25 g KOH-Pulver und 0,5 g Triethylbenzylammoniumchlorid hinzu und tropft 24 g (22 mmol, 65 %ig) Chlormethylpyrazin (J.Org.Chem. 38, 2049 (1973) hinzu. Man rührt über Nacht bei Raumtemperatur, saugt die Salze ab, wäscht das Filtrat mit Kochsalzlösung, trocknet die organische Phase über Kaliumcarbonat, engt ein und destilliert.

Ausbeute: 10,5 g (44 % der Theorie)

Siedepunkt: 126°C/0,4 mbar

Eine gaschromatographische Analyse zeigt, daß das Produkt 84 %ig ist. Daneben enthält es 10 % 5,7-Dihydro-6H-pyrrolo[3,4-b]pyridin-6-carbonsäureethylester und 6 % 2,3-Dihydro-1H-pyrrolo[3,4-c]pyridin-2-carbonsäureethylester.

b) 2,3-Dihydro-1H-pyrrolo[3,4-c]pyridin-2-carbonsäureethylester

Man erhitzt 10,5 g (N-Ethoxycarbony-N-propargyl)-aminomethylpyrazin in 50 ml Trifluoressigsäure 15 Stunden unter Rückfluß. Man gießt den Ansatz auf Wasser, stellt mit $K_2CO_3$ alkalisch, extrahiert mit Methylenchlorid, trocknet die organischen Lösungen über $MgSO_4$, engt ein und kristallisiert aus Waschbenzin um.
Ausbeute: 6,3 g (67,8 % der Theorie)
Schmelzpunkt: 103 ° C
Das Produkt enthält gemäß gaschromatographischer Analyse 6,8 % des isomeren 5,7-Dihydro-6H-pyrrolo[3,4-b]pyridin-6-carbonsäureethylesters.

Die zwei Isomeren können durch Chromatographie an Silicagel mit Essigsäureethylester getrennt werden.

c) 2,3-Dihydro-1H-pyrrolo[3,4-c]pyridin

Man erhitzt 6 g (31,2 mmol) 2,3-Dihydro-1H-pyrrolo[3,4-c]pyridin-2-carbonsäureethylester mit 19,7 g (62,4 mmol) $Ba(OH)_2 \cdot 8H_2O$ in 100 ml Wasser 15 Stunden unter Rückfluß. Nach dem Abkühlen saugt man das $BaCO_3$ ab, engt das Filtrat ein und kocht den Rückstand fünfmal mit je 50 ml Dioxan aus. Die Dioxanlösungen werden eingeengt, der Rückstand destilliert.
Ausbeute: 2,3 g
Siedepunkt: 73 ° C/0,18 mbar
Das Produkt enthält nach $^1$H-NMR 12 % 5,7-Dihydro-6H-pyrrolo[3,4-b]pyridin.

d) 2,3-Dihydro-1H-pyrrolo[3,4-c]pyridin-Dihydrochlorid

Man erhitzt 10,4 g (51 mmol) 2,3-Dihydro-1H-pyrrolo[3,4-c]pyridin-2-carbonsäureethylester mit 100 ml konzentrierter Salzsäure 15 Stunden unter Rückfluß. Man engt den Ansatz ein und verrührt den kristallinen Rückstand mit Aceton. Man saugt ab und trocknet das Produkt an der Luft.
Ausbeute: 9,8 g (100 % der Theorie)

## Beispiel Z3

### 5-Methyl-2,3,4,5,6,7-hexahydro-1H-pyrrolo[3,4-c]pyridin

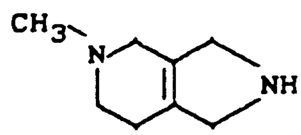

a) 2-Ethoxycarbonyl-5-methyl-2,3-dihydro-1H-pyrrolo[3,4-c]pyridiniumiodid

Man erhitzt 9,6 g (50 mmol) 2,3-Dihydro-1H-pyrrolo[3,4-c]pyridin-2-carbonsäureethylester mit 6,3 ml (100 mmol) Methyliodid in 50 ml Acetonitril 15 Stunden unter Rückfluß. Man gießt den Ansatz in Diethylether, saugt das ausgefallene Salz ab und trocknet es an der Luft.
Ausbeute: 15,6 g (93 % der Theorie)
Schmelzpunkt: 137 - 138 ° C

b) 5-Methyl-2,3,4,5,6,7-hexahydro-1H-pyrrolo[3,4-c]pyridin-2-carbonsäureethylester

Man löst 15,3 g (45,8 mmol) 2-Ethoxycarbonyl-5-methyl-2,3-dihydro-1H-pyrrolo[3,4-c]pyridiniumiodid in 100 ml absolutem Methanol, kühlt auf 0 ° C un$_d$ setzt 7 g (0,1 mol) Natriumborhydrid in 0,5 g-Portionen hinzu. Man rührt anschließend 2 Stunden bei Raumtemperatur, setzt 100 ml Wasser hinzu, versetzt mit $K_2CO_3$ und extrahiert mit $CHCl_3$. Man trocknet die organischen Lösungen über $K_2CO_3$, engt ein und destilliert den Rückstand.
Ausbeute: 7 g (73 % der Theorie)
Siedepunkt: 110 ° C/0,35 mbar

c) 5-Methyl-2,3,4,5,6,7-hexahydro-1H-pyrrolo[3,4-c]pyridin

Man erhitzt 6,3 g (30 mmol) 5-Methyl-2,3,4,5,6,7-hexahydro-1H-pyrrolo[3,4-c]pyridin-2-carbonsäureethylester mit 18,9 g (60 mmol) $Ba(OH)_2 \cdot 8H_2O$ in 75 ml Wasser 15 Stunden unter Rückfluß. Nach dem Abkühlen saugt man das $BaCO_3$ ab, engt das Filtrat ein und kocht den Rückstand fünfmal mit je 50 ml Dioxan aus. Die Dioxanlösungen werden eingeengt und der Rückstand destilliert.
Ausbeute: 2,1 g (46,6 % der Theorie)
Siedepunkt: 95 ° C/10 mbar

## Beispiel Z4

### 2-(N-Ethoxycarbonyl-N-propargyl-aminomethyl)-pyrimidin

a) 2-Aminomethylpyrimidin

66,2 g (0,63 mol) 2-Cyanopyrimidin (Liebigs Ann. Chem. 1981, 333) in 1,9 l Ethanol werden in Gegenwart von 130 ml flüssigem Ammoniak und 5 g Pd-C (5 % Pd) bei 20 ° C und 5 bis 10 bar Wasserstoffdruck hydriert. Der Katalysator wird abfiltriert, das Filtrat aufkonzentriert, und der Rückstand wird destilliert.

Ausbeute: 48,8 g (71 % der Theorie)

Siedepunkt: 82 ° C/4 mbar.

b) 2-Ethoxycarbonylaminomethyl-pyrimidin

49,5 g (0,49 mol) Triethylamin werden zu einer Lösung von 49,5 g (0,45 mol) 2-Aminomethylpyrimidin in 450 ml Toluol gegeben. Anschließend werden 52 g (0,48 mol) Chlorameisensäureethylester tropfenweise unter Eiskühlung zugegeben. Die Mischung wird anschließend 2 Stunden bei Raumtemperatur gerührt. Das entstehende Triethylamin-hydrochlorid wird abgenutscht, das Filtrat mit Kochsalzlösung gewaschen, mit Magnesiumsulfat getrocknet, konzentriert und destilliert.

Ausbeute: 63,3 g (77,6 % der Theorie)

Siedepunkt: 126 ° C/0,09 mbar.

c) 2-(N-Ethoxycarbonyl-N-propargyl-aminomethyl)-pyrimidin

18,1 g (0,1 mol) 2-Ethoxycarbonylaminomethyl-pyrimidin werden tropfenweise zu einer Suspension von 20 g (0,3 mol) pulverisiertem Kaliumhydroxid und 1,1 g (5 mmol) Triethylbenzylammoniumbromid in 200 g Toluol gegeben. Anschließend tropft man 18 g (0,12 mol) Propargylbromid (80 %ige Lösung in Toluol) bei Raumtemperatur zu. Die Mischung wird anschließend während 15 Stunden bei Raumtemperatur gerührt, die Salze werden abgesaugt, das Filtrat mit Kochsalzlösung gewaschen, mit Magnesiumsulfat getrocknet, konzentriert und destilliert. Das Reaktionsprodukt ist mit dem gemäß Beispiel Z1 b) erhaltenen identisch.

Ausbeute: 18 g (86 % der Theorie)

Siedepunkt: 138 ° C/0,8 mbar.

Beispiel 1

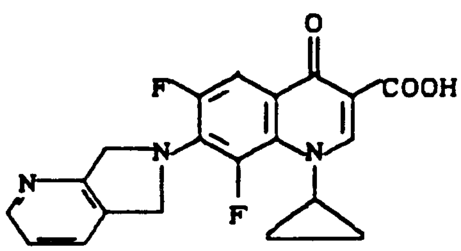

x HCl

A. 1,45 g (5 mmol) 1-Cyclopropyl-6,7,8-trifluor-1,4-dihydro-4-oxo-3-chinolincarbonsäure werden in einer Mischung aus 10 ml Acetonitril und 5 ml Dimethylformamid mit 560 mg (5 mmol) 1,4-Diazabicyclo[2.2.2]-octan und 630 mg (5,3 mmol) 5,7-Dihydro-6H-pyrrolo[3,4-b]pyridin 1 Stunde unter Rückfluß erhitzt. Die Mischung wird eingeengt, der Rückstand mit Wasser verrührt (pH = 7), der ausgefallene Niederschlag abgesaugt und bei 100 ° C im Vakuum getrocknet.

Ausbeute: 1,8 g 1-Cyclopropyl-7-(5,7-dihydro-6H-pyrrolo[3,4-b]pyridin-6-yl)-6,8-difluor-1,4-dihydro-4-oxo-3-chinolincarbonsäure

B. 1,7 g (4,4 mmol) des Produktes aus Stufe A werden in 20 ml halbkonzentrierter Salzsäure gelöst, die Lösung filtriert und das Hydrochlorid durch Zugabe von Ethanol ausgefällt. Das Salz wird abgesaugt und bei 100 ° C im Vakuum getrocknet.

Ausbeute: 1,65 g (89 % der Theorie) 1-Cyclopropyl-7-(5,7-dihydro-6H-pyrrolo[3,4-b]pyridin-6-yl)-6,8-difluor-1,4-dihydro-4-oxo-3-chinolincarbonsäure-Hydrochlorid

Schmelzpunkt: 280 - 290 ° C (unter Zersetzung)

Beispiel 2

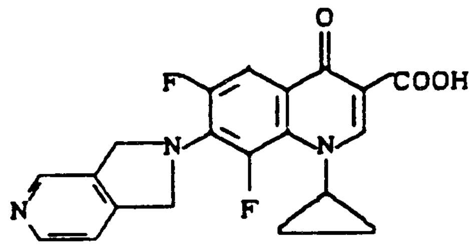

Analog Beispiel 1 A wird mit 2,3-Dihydro-1H-pyrrolo[3,4-c]pyridin (86 %ig mit einem Gehalt von 12 % des isomeren 5,7-Dihydro-6H-pyrrolo[3,4-b]pyridins) zu 1-Cyclopropyl-7-(2,3-dihydro-1H-pyrrolo[3,4-c]-pyridin-2-yl)-6,8-difluor-1,4-dihydro-4-oxo-3-chinolincarbonsäure umgesetzt, das durch etwa 10 % des isomeren Produktes aus Beispiel 1 A verunreinigt ist.
Schmelzpunkt: 246 - 249 ° C (unter Zersetzung) (umkristallisiert aus Dimethylformamid)

Beispiel 3

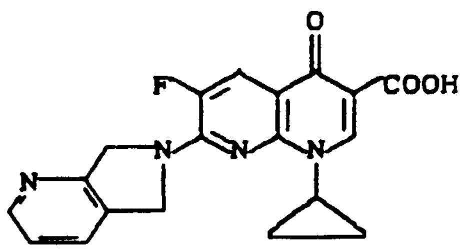

282 mg (1 mmol) 7-Chlor-1-cyclopropyl-6-fluor-1,4-dihydro-4-oxo-1,8-naphthyridin-3-carbonsäure werden in 3 ml Acetonitril mit 240 mg (2 mmol) 5,7-Dihydro-6H-pyrrolo[3,4-b]pyridin1 Stunde bei Raumtemperatur gerührt. Der ungelöste Feststoff wird abgesaugt, mit Acetonitril und Wasser gewaschen und bei 100 ° C im Hochvakuum getrocknet.
Ausbeute: 170 mg (46 % der Theorie) 1-Cyclopropyl-(5,7-dihydro-6H-pyrrolo[3,4-b]pyridin-6-yl)-6-fluor-1,4-dihydro-4-oxo-1,8-naphthyridin-3-carbonsäure
Schmelzpunkt: 275-280 ° C (unter Zersetzung)

Beispiel 4

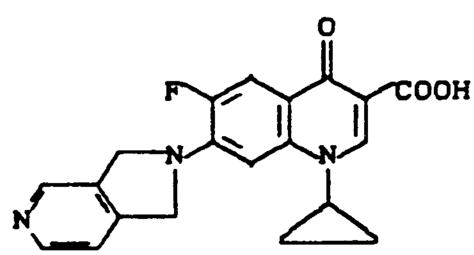

1,33 g (5 mmol) 1-Cyclopropyl-6,7-difluor-1,4-dihydro-4-oxo-3-chinolincarbonsäure werden in einer Mischung aus 10 ml Acetonitril und 5 ml Dimethylformamid in Gegenwart von 1,8 g (1,6 mmol) 1,4-Diazabicyclo[2.2.2]octan und 1,7 (9 mmol) 2,3-Dihydro-1H-pyrrolo[3,4-c]pyridin-Hydrochlorid 5 Stunden unter Rückfluß erhitzt. Die Suspension wird abgekühlt, der Niederschlag abgesaugt, mit etwa 50 ml Wasser gewaschen und bei 100 ° C im Vakuum getrocknet.
Ausbeute: 1,52 g (83 % der Theorie) 1-Cyclopropyl-7-(2,3-dihydro-1H-pyrrolo[3,4-c]pyridin-2-yl)-6-fluor-1,4-dihydro-4-oxo-3-chinolincarbonsäure,

Schmelzpunkt: 297 - 300 ° C (unter Zersetzung).

Beispiel 5

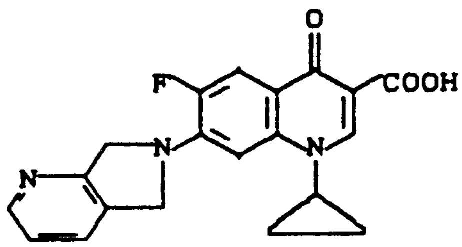

Analog Beispiel 4 wird mit 5,7-Dihydro-6H-pyrrolo[3,4-b]pyridin-Hydrochlorid zu 1-Cyclopropyl-7-(5,7-dihydro-6H-pyrrolo[3,4-b]pyridin-6-yl)-6-fluor-1,4-dihydro-4-oxo-3-chinolincarbonsäure vom Schmelzpunkt 300 -304 ° C (unter Zersetzung) umgesetzt.

Beispiel 6

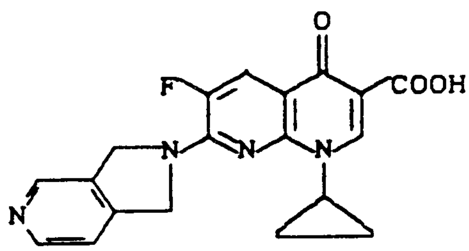

Analog Beispiel 3 wird mit 2,3-Dihydro-1H-pyrrolo[3,4-c]pyridin (86 %ig) zu 1-Cyclopropyl-7-(2,3-dihydro-1H-pyrrolo[3,4-c]pyridin-2-yl]-6-fluor-1,4-dihydro-4-oxo-1,8-naphthyridin-3-carbonsäure vom Schmelzpunkt 275 - 280 ° C (unter Zersetzung) umgesetzt.

Beispiel 7

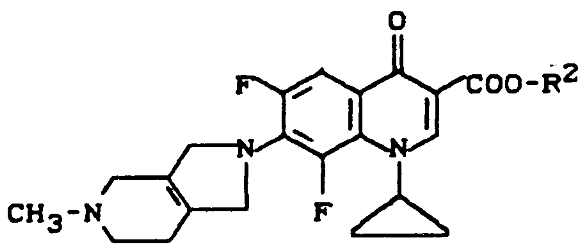

A. $R^2 = C_2H_5$

B. $R^2 = H \times HCl$

C. $R^2 = H$

A. Eine Lösung von 850 mg (2,7 mmol) 1-Cyclopropyl-6,7,8-trifluor-1,4-dihydro-4-oxo-3-chinolincarbonsäure-ethylester in 5 ml Dimethylformamid und 10 ml Acetonitril wird mit 370 mg (3,3 mmol) 1,4-Diazabicyclo[2.2.2]octan und 570 mg (3,8 mmol) 5-Methyl2,3,4,5,6,7-hexahydro-1H-pyrrolo-[3,4-c]pyridin 7 Stunden unter Rückfluß erhitzt. Die Mischung wird eingedampft, der Rückstand mit Wasser verrührt, das ungelöste Produkt abgesaugt, mit Wasser gewaschen und bei 100 ° C im Vakuum getrocknet.
Ausbeute: 790 mg (68 % der Theorie) 1-Cyclopropyl-6,8-difluor-1,4-dihydro-7-(5-methyl-2,3,4,5,6,7-hexahydro-1H-pyrrolo[3,4-c]pyridin-2-yl)-4-oxo-3-chinolincarbonsäure-ethylester
Schmelzpunkt: 163 - 165 ° C (unter Zersetzung) (aus Glykolmonomethylether)

B. 500 mg (1,2 mmol) des Produktes aus Stufe A werden in einer Mischung aus 4 ml Eisessig und 3 ml konzentrierter Salzsäure 2 Stunden unter Rückfluß erhitzt. Die Mischung wird eingeengt, der Rückstand mit Ethanol verrührt, das Hydrochlorid abgesaugt, mit Ethanol gewaschen und bei 100 °C im Vakuum getrocknet.
Ausbeute: 180 mg (35 % der Theorie) 1-Cyclopropyl-6,8-difluor-1,4-dihydro-7-(5-methyl-2,3,4,5,6,7-hexahydro-1H-pyrrolo[3,4-c]pyridin-2-yl)-4-oxo-3-chinolincarbonsäure-Hydrochlorid
Schmelzpunkt: 274 - 275 °C (unter Zersetzung)
C. 850 mg (3 mmol) 1-Cyclopropyl-6,7,8-trifluor-1,4-dihydro-4-oxo-3-chinolincarbonsäure werden unter den Bedingungen von Beispiel 19 A umgesetzt.
Ausbeute: 1 g (83 % der Theorie) 1-Cyclopropyl-6,8-difluor-1,4-dihydro-7-(5-methyl-2,3,4,5,6,7-hexahydro-1H-pyrrolo[3,4-c]pyridin-2-yl)-4-oxo-3-chinolincarbonsäure
Schmelzpunkt: 210 - 213 °C (unter Zersetzung)
Durch Lösen dieses Beteins in halbkonzentrierter Salzsäure, Eindampfen der Lösung im Vakuum und Verrühren des Rückstandes mit Ethanol erhält man das Hydrochlorid, das mit dem Produkt aus Beispiel 19 B identisch ist.

Beispiel 8

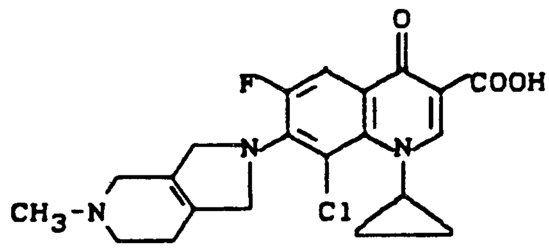

900 mg (3 mmol) 8-Chlor-1-cyclopropyl-6,7-difluor-1,4-dihydro-4-oxo-3-chinolincarbonsäure werden in einer Mischung aus 10 ml Acetonitril und 5 ml Dimethylformamid mit 370 mg (3,3 mmol) 1,4-Diazabicyclo-[2.2.2]octan und 495 mg (3,3 mmol) 5-Methyl-2,3,4,5,6,7-hexahydro-1H-pyrrolo[3,4-c]pyridin 1 Stunde unter Rückfluß erhitzt.

Die Suspension wird gekühlt, der Niederschlag abgesaugt, mit Wasser gewaschen und bei 80 °C im Vakuum getrocknet.
Ausbeute: 1 g (80 % der Theorie) 8-Chlor-1-cyclopropyl-6-fluor-1,4-dihydro-7-(5-methyl-2,3,4,5,6,7-hexahydro-1H-pyrrolo[3,4-c]pyridin-2-yl)-4-oxo-3-chinolincarbonsäure
Schmelzpunkt: 215 - 217 °C (unter Zersetzung) (umkristallisiert aus Acetonitril)

Beispiel 9

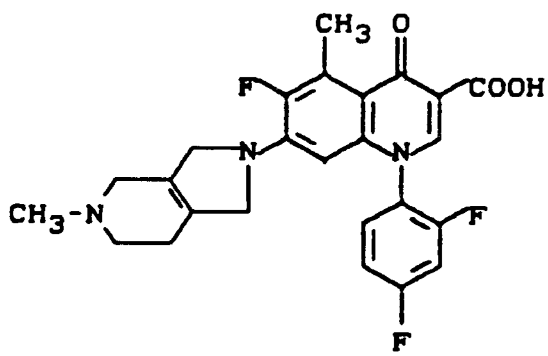

Analog Beispiel 7 A wird mit 1-(2,4-Difluorphenyl)-6,7-difluor-1,4-dihydro-5-methyl-4-oxo-3-chinolincarbonsäure zu 1-(2,4-Difluorphenyl)-6-fluor-1,4-dihydro-5-methyl-7-(5-methyl-2,3,4,5,6,7-hexahydro-1H-pyrrolo-[3,4-c]pyridin-2-yl)-4-oxo-3-chinolincarbonsäure vom Schmelzpunkt 270 - 273 °C (unter Zersetzung) umgesetzt.

**Patentansprüche**

**1.** Chinolin- und Naphthyridoncarbonsäure-Derivate der Formel (I)

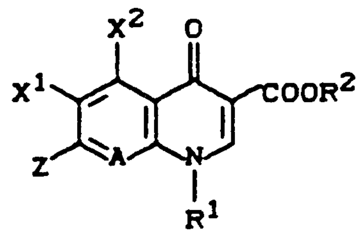

(I),

in welcher

- $X^1$ für Halogen,
- $X^2$ für Wasserstoff, Amino, Alkylamino mit 1 bis 4 Kohlenstoffatomen, Dialkylamino mit 1 bis 3 Kohlenstoffatomen je Alkylgruppe, Hydroxy, Alkoxy mit 1 bis 4 Kohlenstoffatomen, Mercapto, Alkylthio mit 1 bis 4 Kohlenstoffatomen, Arylthio, Halogen oder Methyl,
- $R^1$ für Alkyl mit 1 bis 4 Kohlenstoffatomen, Alkenyl mit 2 bis 4 Kohlenstoffatomen, Cycloalkyl mit 3 bis 6 Kohlenstoffatomen, 2-Hydroxyethyl, 2-Fluorethyl, Methoxy, Amino, Methylamino, Ethylamino, Dimethylamino, gegebenenfalls durch 1 oder 2 Fluoratome substituiertes Phenyl,
- $R^2$ für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-methyl,
- Z für einen Rest der Struktur

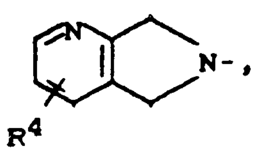

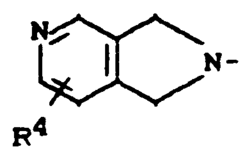

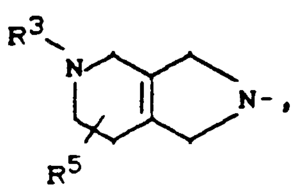

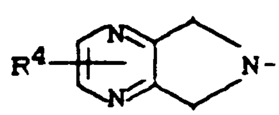

steht, worin

- $R^3$ für Wasserstoff, gegebenenfalls durch Hydroxy substituiertes $C_1$-$C_3$-Alkyl, Alkoxycarbonyl mit 1 bis 4 C-Atomen im Alkoxyteil oder $C_1$-$C_3$-Acyl,
- $R^4$ für Wasserstoff, Hydroxy,

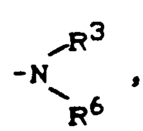

Hydroxymethyl oder

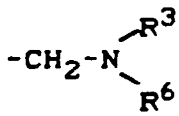

steht, wobei

$R^6$ Wasserstoff oder Methyl bedeutet,

$R^5$ für Wasserstoff, $C_1$-$C_3$-Alkyl oder Cyclopropyl und

A für N oder C-$R^7$ steht, worin

$R^7$ für H, Halogen, Methyl, Hydroxy oder Methoxy steht oder auch gemeinsam mit $R^1$ eine Brücke der Struktur

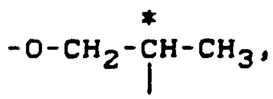 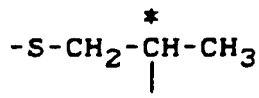 oder

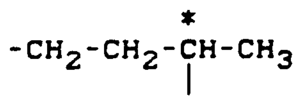

bilden kann, mit der Maßgabe, daß A nicht N, CH oder CF sein kann und mit $R^1$ keine Brücke

der Struktur

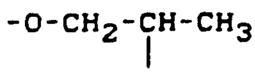

bilden kann, wenn

Z für

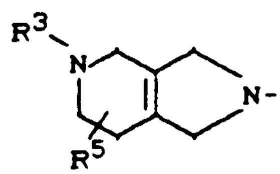

steht,

und deren pharmazeutisch verwendbaren Hydrate und Säureadditionssalze sowie Alkali-, Erdalkali-, Silber- und Guanidiniumsalze.

**2.** Verbindungen gemäß Anspruch 1 der Formel (I),

in welcher

$X^1$ für Fluor,

$X^2$ für Wasserstoff, Amino, Methylamino, Hydroxy, Methoxy, Fluor, Chlor, Brom oder Methyl,

$R^1$ für Alkyl mit 1 bis 3 Kohlenstoffatomen, Vinyl, Cycloalkyl mit 3 bis 4 Kohlenstoffatomen, 2-Fluorethyl, gegebenenfalls durch 1 oder 2 Fluoratome substituiertes Phenyl,

$R^2$ für Wasserstoff, gegebenenfalls durch Amino, Methylamino oder Dimethylamino substituiertes Alkyl mit 1 bis 2 Kohlenstoffatomen oder (5-Methyl-2-oxo-1,3-dioxol-4-yl)-methyl,

Z für einen Rest der Struktur

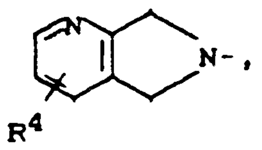 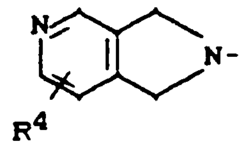

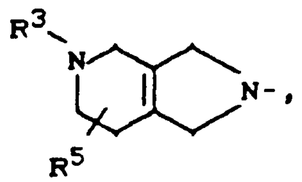 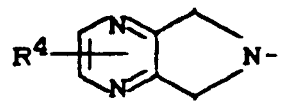

steht, worin

$R^3$ für Wasserstoff, gegebenenfalls durch Hydroxy substituiertes $C_1$-$C_2$-Alkyl, Alkoxycarbonyl mit 1 bis 4 C-Atomen im Alkoxyteil oder $C_1$-$C_3$-Acyl,

$R^4$ für Wasserstoff, Hydroxy oder

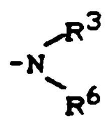

steht, wobei

$R^6$ Wasserstoff oder Methyl bedeutet,

$R^5$ für Wasserstoff oder Methyl und

A für N oder C-$R^7$ steht, worin

$R^7$ für H, Fluor, Chlor, Brom, Methyl oder Methoxy steht oder auch gemeinsam mit $R^1$ eine Brücke der Struktur

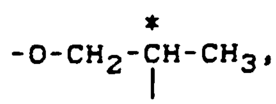

bilden kann, mit der Maßgabe, daß A nicht N, CH oder CF sein kann und mit $R^1$ keine Brücke

der Struktur

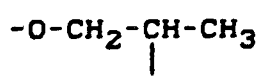

bilden kann, wenn

Z für

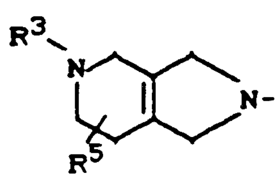

steht,

und deren pharmazeutisch verwendbare Hydrate und Säureadditionssalze sowie Alkali-, Erdalkali-, Silber- und Guanidiniumsalze.

**3.** Verbindungen gemäß Anspruch 1 der Formel (I),

in welcher

$X^1$ für Fluor,

$X^2$ für Wasserstoff, Amino, Fluor oder Brom,

$R^1$ für Alkyl mit 1 bis 2 Kohlenstoffatomen, Cyclopropyl, gegebenenfalls durch 1 oder 2 Fluoratome substituiertes Phenyl,

$R^2$ für Wasserstoff oder Alkyl mit 1 bis 2 Kohlenstoffatomen,

Z für einen Rest der Struktur

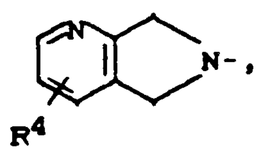 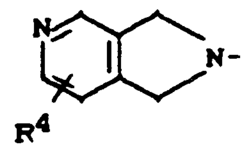

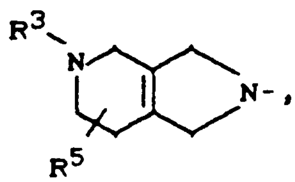 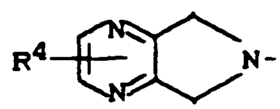

steht, worin

$R^3$ für Wasserstoff, Methyl, Alkoxycarbonyl mit 1 bis 4 C-Atomen im Alkoxyteil oder $C_1$-$C_3$-Acyl,

$R^4$ für Wasserstoff, Hydroxy oder

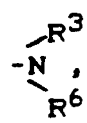

steht, wobei

$R^6$ Wasserstoff oder Methyl bedeutet,

$R^5$ für Wasserstoff oder Methyl und

A für N oder C-$R^7$ steht, worin

$R^7$ für H, Fluor, Chlor oder Methoxy steht oder auch gemeinsam mit $R^1$ eine Brücke der Struktur

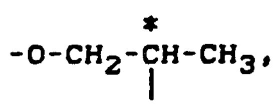

bilden kann, mit der Maßgabe, daß A nicht N, CH oder CF sein kann und mit $R^1$ keine Brücke der Struktur

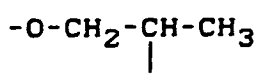

bilden kann, wenn

Z für

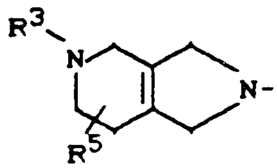

steht,
und deren pharmazeutisch verwendbaren Hydrate und Säureadditionssalze sowie Alkali-, Erdalkali-, Silber- und Guanidiniumsalze.

**4.** 8-Chlor-1-cyclopropyl-6-fluor-1,4-dihydro-7-(5-methyl-2,3,4,5,6,7-hexahydro-1H-pyrrolo[3,4-c]pyridin-2-yl)-4-oxo-3-chinolincarbonsäure.

**5.** 1-(2,4-Difluorphenyl)-6-fluor-1,4-dihydro-5-methyl-7-(5-methyl-2,3,4,5,6,7-hexahydro-1H-pyrrolo[3,4-c]-pyridin-2-yl)-4-oxo-3-chinolincarbonsäure.

**6.** 5,7-Dihydro-6H-pyrrolo[3,4-b]pyridin.

**7.** 2,3-Dihydro-1H-pyrrolo[3,4-b]pyrazin.

**8.** 2-(N-Ethoxycarbonyl-N-propargylaminomethyl)-pyrimidin.

**9.** 5,7-Dihydro-6H-pyrrolo[3,4-b]pyridin-6-carbonsäure.

**10.** (N-Ethoxycarbonyl-N-propargylaminomethyl)pyrazin.

**11.** Verfahren zur Herstellung von Verbindungen gemäß Anspruch 1 der Formel (I), dadurch gekennzeichnet, daß man Verbindungen der Formel (II)

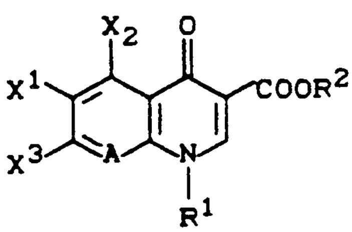

(II),

in welcher
A, $R^1$, $R^2$, $X^1$ und $X^2$ die oben angegebene Bedeutung haben und
$X^3$ für Halogen, insbesondere Fluor oder Chlor steht,
mit Verbindungen der Formel (III)

Z-H (III)

in welcher
Z die oben angegebene Bedeutung hat,
gegebenenfalls in Gegenwart von Säurefängern umsetzt.

**12.** Arzneimittel enthaltend Verbindungen gemäß Ansprüchen 1 bis 5 .

**13.** Verwendung von Verbindungen gemäß Ansprüchen 1 bis 5 bei der Herstellung von Arzneimitteln.